# EUROPEAN PATENT SPECIFICATION

(11) **EP 3 446 124 B1**
(45) Date of publication and mention of the grant of the patent: **05.08.2026**
(21) Application number: 17718062.7
(22) Date of filing: 18.04.2017
(51) Int. Cl.: G01N 33/68

(54) **SOLUBLE ST2 FOR THE IDENTIFICATION OF PROGRESSORS TO LVH IN THE GENERAL POPULATION**
LÖSLICHES ST2 ZUR IDENTIFIZIERUNG VON PROGRESSOREN VON LVH IN DER GESAMTBEVÖLKERUNG
ST2 SOLUBLE POUR L'IDENTIFICATION DE PROGRESSEURS VERS HVG DANS LA POPULATION GÉNÉRALE

(30) Priority: 18.04.2016 EP 16165776
(43) Date of publication of application: 27.02.2019
(73) Proprietor: Roche Diagnostics GmbH, 68305 Mannheim (DE); F. Hoffmann-La Roche AG, 4070 Basel (CH); Centre Hospitalier Régional et Universitaire de Nancy, 54000 Nancy (FR)
(72) Inventor: BLOCK, Dirk, 83673 Bichl (DE); WIENHUES-THELEN, Ursula-Henrike, 82152 Krailling (DE); ZAUGG, Christian, 4310 Rheinfelden (CH); ZIEGLER, André, 4448 Laeufelfingen (CH); ZANNAD, Faiez, 54600 Villers Les Nancy (FR); ROSSIGNOL, Patrick, 54000 Nancy (FR)
(74) Representative: Altmann Stößel Dick Patentanwälte PartG mbB
(86) International application number: PCT/EP2017/059137
(87) International publication number: WO 2017/182446

(56) References cited:
- EP-A1- 2 478 371
- NAKAMURA HIRONORI ET AL: "Novel Markers of Left Ventricular Hypertrophy in Uremia", AMERICAN JOURNAL OF NEPHROLOGY, vol. 31, no. 4, 1 January 2010 (2010-01-01), CH, pages 292 - 302, XP55773617, ISSN: 0250-8095, Retrieved from the Internet <URL:https://www.ncbi.nlm.nih.gov/pmc/articles/PMC2924237/pdf/ajn0031-0292.pdf> DOI: 10.1159/000279768
- D B OJJI ET AL: "The effect of left ventricular remodelling on soluble ST2 in a cohort of hypertensive subjects", JOURNAL OF HUMAN HYPERTENSION, vol. 28, no. 7, 9 January 2014 (2014-01-09), GB, pages 432 - 437, XP055293492, ISSN: 0950-9240, DOI: 10.1038/jhh.2013.130
- DIKE B. OJJI ET AL: "Relationship Between Left Ventricular Geometry and Soluble ST2 in a Cohort of Hypertensive Patients", THE JOURNAL OF CLINICAL HYPERTENSION, vol. 15, no. 12, 30 December 2013 (2013-12-30), US, pages 899 - 904, XP055293608, ISSN: 1524-6175, DOI: 10.1111/jch.12205
- SINTEK MARC ET AL: "GDF15 AND ST2 IDENTIFY PATIENTS WITH AORTIC STENOSIS AND SEVERE LEFT VENTRICULAR HYPERTROPHY AT INCREASED RISK FOR MORTALITY AFTER AORTIC VALVE REPLACEMENT", JOURNAL OF THE AMERICAN COLLEGE OF CARDIOLOGY, ELSEVIER, NEW YORK, NY, US, vol. 67, no. 13, Suppl, 1 April 2016 (2016-04-01), pages 2195, XP009191254, ISSN: 0735-1097

## Description

The present invention relates to methods and uses as specified in the appended claims
Heart failure (HF) is a major and growing public health problem. It is estimated that approximately 5 million patients in the USA have HF, more than 500 000 patients are diagnosed with HF for the first time each year, and more than 250 000 patients in the USA die each year of HF as a primary cause. HF is one of the main causes of morbidity and mortality in developed countries. Because of aging of the population and greater longevity of patients with cardiovascular diseases incidence and prevalence of HF are increasing.

HF often starts with a change in the geometry of the left ventricle, changing potentially both systolic and diastolic function (left ventricular dysfunction LVD). LVD is sub-divided into systolic LVD and diastolic LVD. LVD may develop into left ventricular hypertrophy (LVH) in which the walls of the ventricle thicken.

Hypertrophy as a response to the increased afterload associated with elevated systemic vascular resistance is necessary and protective up to a certain point. Beyond that point, a variety of dysfunctions accompany LVH, including lower coronary vasodilatory capacity, depressed left ventricular wall mechanics, and abnormal left ventricular diastolic filling pattern. LVH finally may lead to chronic HF with the final stage of terminal HF.

It is desirable to identify risk subjects as early as possible. In particular, it is of importance to identify those subjects who are at risk of progressing to LVH as early as possible since appropriate preventive measures can be initiated before progression to LVH (see e.g. Diez and Frohlich; Hypertension. 2010;55:1-8). Preventive measures e.g. comprise lifestyle changes and administration of antihypertensives according to guidelines to prevent progression to LVH in particular in younger subjects.

ST2, also known as "Interleukin 1 receptor-like 1" is a member of the IL-1 receptor family that is produced by cardiac fibroblasts and cardiomyocytes under conditions of mechanical stress. ST2 is an interleukin-1 receptor family member and exists in a membrane-bound isoform and a soluble isoform (sST2).

Soluble ST2 has been proposed as a cardiac biomarker. E.g., elevated soluble ST2 has been associated with a worse prognosis in patients having already existing cardiovascular diseases (Weinberg EO et al. Circulation 2003;107:721-726, Sabatine MS et al. Circulation 2008;117:1936-1944). Also, effects of left ventricular remodelling on soluble ST2 in hypertensive subjects were reported (Ojji et al., Journal of Human Hypertension (2014) 28, 432-437; Ojji et al., The Journal of Clinical Hypertension (2013) 15(12), 899), Moreover, GDF15 and ST2 were reported to identify patients with aortic stenosis and severe left ventricular hypertrophy at increased risk for mortality after aortic valve replacement (Sintek et al., Journal of the A american College of Cardiology (2016) 67(13), Suppl, 2195).

In three community-based studies soluble ST2 has been examined with regard to cardiovascular outcomes and all cause mortality.

In the Framingham Heart Study, the participants were followed over a mean of 11.3 years. Elevated concentrations of soluble ST2 were found to be predictive for incident heart failure and all cause mortality (Wang TJ et al. Circulation 2012;126:1596-1604).

In the Dallas Heart Study baseline soluble ST2 was found predictive for all cause mortality. Study participants were followed over a mean of 8.3 years (Chen LQ et al. Clin Chem 2013;59:536-546).

In 3,915 participants of the Cardiovascular Health Study followed over a mean of 13.6 years the concentration of soluble ST2 at baseline predicted incident HF events (low ejection fraction) and cardiovascular death (Ginsberg E. J Am Coll Cardiol 2014; 63: A768).

Soluble ST2 has been also proposed as a marker for therapy guidance. Weir et al. found an association between ST2 and aldosterone levels and propose to use ST2 for selection of patients for aldosterone antagonist therapy in patients after myocardial infarction (Weir RA et al. J Am Coll, Cardiol. 2010 Jan 19;55(3):243-50).

Ho et al. reviews scientific literature dealing with soluble ST2 testing in the general population (Am J Cardiol 2015;115[suppl]:22B-25B). The document is focused on late cardiovascular outcomes (such as mortality). Further, it does not deal with left ventricular hypertrophy. The authors state that there may be a role for soluble ST2 in improving current risk-stratification strategies for the prediction of cardiovascular outcomes. However, the role of soluble ST2 testing in the general population has not yet been conclusively established.

Soluble ST2 has not been shown to be associated as a risk predictor for the development of Left Ventricular Hypertrophy (LVH). So far there is no reliable method available to early predict the risk of individuals that may progress to LVH. Thus, means and measures for identifying a subject being at risk of progressing to LVH are highly required. The present invention provides such means and measures.

Advantageously, it has been shown in the context of the studies underlying the present invention that the determination of soluble ST2 in a sample from a subject allows for the reliable identification of individuals in the general population being at risk of progression to LVH. In particular, the method allows for the identification of individuals at risk long before onset of clinical symptoms and not only in the elderly population but advantageously also in younger individuals.

Accordingly, the present invention relates to a method for identifying a subject who is at risk of progressing to left ventricular hypertrophy (LVH) according to claim 1; further disclosure relates to a method for identifying a subject who is at risk of progressing to LVH, comprising the steps of
(a) measuring the amount of the biomarker soluble ST2 in a sample from the subject, and
(b) comparing the amount of soluble ST2 to a reference.

According to the present invention, a subject who is at risk of progressing to left ventricular hypertrophy is identified by carrying out the further step (c) of identifying a subject who is at risk of progressing to left ventricular hypertrophy. Said identification shall be based on the result of the comparison carried out in step (b).

Moreover, the present invention relates to a method for predicting the risk of a subject of progressing to left ventricular hypertrophy (LVH) according to claim 7; further disclosure relates to a method for predicting the risk of a subject of progressing to LVH, comprising the steps of
(a) measuring the amount of the biomarker soluble ST2 in a sample from the subject,
(b) comparing the amount of soluble ST2 to a reference, and optionally
(c) predicting the risk of the subject of progressing to LVH based on the results of step (b).

The method of the present invention is an in vitro method. Moreover, it may comprise steps in addition to those explicitly mentioned above. For example, further steps may relate to sample pre-treatments or evaluation of the results obtained by the method.

The method of the present invention may be also used for monitoring, confirmation, and sub-classification of the subject. The method may be carried out manually or assisted by automation. Preferably, step (a), (b) and/or (c) may in total or in part be assisted by automation, e.g., by a suitable robotic and sensory equipment for the determination in step (a) or a computer-implemented calculation in step (b).

The term "identifying a subject" as used herein preferably refers to using the information or data generated relating to the amount of soluble ST2 in a sample of a subject to identify a subject who is at risk or who is not at risk of progressing to LVH. Preferably, the subject is identified because the subject is at risk (or not at risk) of progressing to LVH. In an embodiment, a subject is identified who is not at risk of progressing to LVH, in particular within a certain window period. The actual identification, prediction or differentiation as referred to the methods or uses of the present invention may comprise further steps such as the confirmation of the identification, prediction or differentiation. Thus, the identification, prediction or differentiation is, preferably, understood as an aid in the identification, prediction or differentiation.

The information or data used or generated may be in any form, written, oral or electronic. In some embodiments, using the information or data generated includes communicating, presenting, reporting, storing, sending, transferring, supplying, transmitting, dispensing, or combinations thereof. In some embodiments, communicating, presenting, reporting, storing, sending, transferring, supplying, transmitting, dispensing, or combinations thereof are performed by a computing device, analyzer unit or combination thereof. The information or data includes a comparison of the amount of the biomarker to a reference amount. In some embodiments, the information or data includes an indication that the subject is more likely or less likely to progress to LVH.

By carrying out the method of the present invention, the subject is allocated either into the group of subjects being at risk of progressing to LVH, or into the group of subjects being not at risk of progressing to LVH. A subject who is at risk, preferably, is a subject who is at elevated risk of progressing to LVH (in particular within a certain predictive window). Preferably, said risk is elevated as compared to the average risk in a cohort of subjects (i.e. a group of subjects). A subject who is not at risk, preferably, is a subject who is at reduced risk of progressing to LVH (in particular within the predictive window). Preferably, said risk is reduced as compared to the average risk in a cohort of subjects (i.e. a group of subjects). Accordingly, the method of the present invention allows for differentiating between an elevated risk or a reduced risk. A subject who is at risk of progressing to LVH preferably has a risk of about 5% or larger, or, more preferably of about 5-12% or larger, or most preferably of about 20% or larger of progressing to LVH, preferably, within a predictive window of about 5 years. A subject who is not at risk preferably has a risk of lower than about 5%, more preferably of lower than, about 4%, or most preferably of lower than about 1% of progressing to LVH, preferably, within a predictive window of about 5 years.

A subject who is at risk of progressing to LVH preferably has a risk of about 5% or larger, or, more preferably of about 5-12% or larger, or most preferably of about 20 % or larger of progressing to LVH, preferably, within a predictive window of about 10 years. A subject who is not at risk preferably has a risk of lower than about 5%, more preferably of lower than, about 4%, or most preferably of lower than about 1% of progressing to LVH, preferably, within a predictive window of about 10 years.

A subject who is at risk of progressing to LVH preferably has a risk of about 5 % or larger, or, more preferably of about 5-12 % or larger, or most preferably of about 20 % or larger of progressing to LVH, preferably, within a predictive window of about 17 years. A subject who is not at risk preferably has a risk of lower than about 5 %, more preferably of lower than, about 4 %, or most preferably of lower than about 1 % of progressing to LVH, preferably, within a predictive window of about 17 years.

As will be understood by those skilled in the art, the identification (or prediction) is usually not intended to be correct for 100% of the subjects. The term, however, requires that the identification can be made for a statistically significant portion of subjects in a proper and correct manner. Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98%, or at least 99%. The p-values are, preferably, 0.1, 0.05, 0.01, 0.005, or 0.0001.

In accordance with the present invention a subject shall be identified who is at risk of progressing to left ventricular hypertrophy (LVH). The term "left ventricular hypertrophy" is well known in the art. As used herein, the term preferably relates to a thickening of the walls of the ventricle. LVH is known to be a response to a chronically increased workload on the heart. LVH found in patients suffering from arterial hypertension (i.e. high blood pressure) is a disease requiring treatment.

A detailed overview on left ventricular hypertrophy can be, e.g. found in standard text books (see Swamy Curr Cardiol Rep (2010) 12:277-282). LVH can be detected by electro-cardiography, echocardiography, or cardiac magnetic resonance imaging (MRI). Preferably, LVH is detected by echocardiography. Moreover, criteria for the diagnosis of LVH are well known in the art (see Mancia et al., European Heart J. 2007, 28: 1462, Die Innere Medizin: Referenzwerk für den Facharzt - Wolfgang Gerok - 2007, page 293., Swamy Curr Cardiol Rep (2010) 12:277-282).

The assessment of the left ventricular mass und thus of LVH, preferably, includes measurements of the septum diameter, left ventricular posterial wall thickness and end diastolic diameter, with calculation of left ventricular mass according to formulae known in the art. Particularly preferred criteria for diagnosing LVH are e.g. disclosed in the guidelines (Mancia et al., European Heart J. 2007, 28: 1462). Also preferably, the criteria may be taken from the ACC/AHA guidelines for the evaluation and management of chronic heart failure in the adult (J. Am. Coll. Cardiol. 2001;38;2101-2113).

In a preferred embodiment, the left ventricular mass is determined by the ASE equation. The ASE equation is a formula for estimation of left ventricular mass and is based on modeling the left ventricle as a prolate ellipse of revolution. The formula is based on the left ventricular end-diastolic diameter, the diastolic posterior wall thickness, and the diastolic septal wall thickness

In an embodiment, the formula of the ASE equation is as follows:
Left ventricular mass = 0.8 x (1.04 x (LVEDD+ PWTd + SWTd)³ - (LVEDD)³)) + 0.6, wherein LVEDD is the left ventricular end-diastolic diameter, PWTd is the diastolic posterior wall thickness, and SWTd is the diastolic septal wall thickness.

This formula is commonly applied on M-mode but can also be applied on 2D measurements in echocardiography.

Also preferably, the ventricular mass can be determined by the following formula:
LV mass = 0.8 x 1.04 [( IVS + LVID + PWT)³- (LVID)³)) + 0.6 g, wherein IVS is the interventricular septum thickness, LVID is the LV internal diameter and PWT is inferolateral wall thickness

Preferably, the left ventricular mass is normalized by body height or in particular by body surface area. The ratio of the left ventricular mass to the body surface area is the Left Ventricular Mass Index (abbreviated LVMI). Preferably, the LVMI is determined as described in the Examples section. Also preferably, the LVMI is determined as disclosed by Lang et al. (Recommendations for chamber quantification. Eur J Echocardiogr 2006;7:79-108).

Preferably, a male subject suffers from LVH if the left ventricular mass index (and, thus, the ratio of the left ventricular mass to body surface area, abbreviated LVMI) is larger than 116 g/m². Preferably, a female subject suffers from LVH, if the LVMI is larger than 96 g/m².

Thus, if the subject to be tested is male, a subject who is at risk of progressing to LVH is a subject who is at risk of progressing to a left ventricular mass index of larger than 116 g/m². Thus, if the subject to be tested is female, a subject who is at risk of progressing to LVH is a subject who is at risk of progressing to a left ventricular mass index of larger than 96 g/m².

In accordance with the present invention, a subject shall be identified who is at risk of progressing to LVH (or the risk of progressing to LVH shall be predicted). The expression "progressing to LVH" is well understood by the skilled person. A subject who is at risk of progressing to LVH, preferably, is a subject who is at risk of progressing to LVH within a certain predictive window (after the sample has been obtained). Preferably, said predictive window is calculated from the time point at which the sample to be tested has been obtained. Preferred predictive windows are given herein below.

In a preferred embodiment, the period is a period of about five years (or less than five years). Thus, a subject is identified who is a risk of progressing to LVH within period of about five years (or of less than five years), or the risk to progress to LVH within a period of about five years (or of less than five years) is predicted.

In another preferred embodiment, the predictive window is a period of about 10 years.

In another preferred embodiment, the predictive window is a period of about 15 years.

In another preferred embodiment, the predictive window is a period of about 17 years.

In another preferred embodiment, the predictive window is a period of about 20 years.

The prediction within an interval of about 3 to about 20 years, in particular of about 5 to about 20 years is considered as prediction of the long term risk.

The prediction within an interval of about one month to about three years (after the sample has been obtained) is considered as prediction of the short term risk. Further, it is envisaged that the predictive window is an interval of about one month to two years, or of one month to about 18 months. Further preferred intervals for the prediction of a short term risk are disclosed elsewhere herein. In an embodiment, the short term risk is predicted by measuring the amount of sST2 and a BNP-type peptide and comparing the thus measured amounts to reference amounts.

Preferably, the term "about" as used herein encompasses a range of + and - 20% relative to the specific value, amount, concentration, level, etc., e.g., indication of a value of "about 100" is meant to encompass a value of a numerical range of 100+/-20%, i.e., a value range from 80 to 120. Preferably, the term encompasses a range of + and -10% relative to the specific value, amount, concentration, level, etc. and, more preferably, a range of + and -5% relative to the specific value, amount, concentration, level, etc. Most preferably, the term "about" refers to the exact value, amount, concentration, level, etc.

The "subject" as referred to herein is, preferably, a mammal. Mammals include, but are not limited to, domesticated animals (e.g., cows, sheep, cats, dogs, and horses), primates (e.g., humans and non-human primates such as monkeys), rabbits, and rodents (e.g., mice and rats). Preferably, the subject is human. As set forth above, the subject may be male or female.

Preferably, the subject to be tested is more than 30 or more than 40 years of age. More preferably, the subject is between 30 and 50 years of age. Even more preferably, the subject is between 40 and 50 years of age. Most preferably the subject is between 40 and 45 years of age.

In the context of the present invention, it is in particular envisaged that the subject to be tested is apparently healthy. A subject who is apparently healthy does not show obvious signs of an underlying disease. In accordance with the present invention a subject who is apparently healthy is, preferably, a subject who does not show symptoms of heart failure. In particular, a subject who is apparently healthy does not suffer from heart failure.

The term "heart failure" as used herein relates to an impaired systolic and/or diastolic function of the heart. Said impaired systolic and/or diastolic function of the heart may or may not be accompanied by overt signs of heart failure as known to the person skilled in the art. Preferably, heart failure referred to herein is also chronic heart failure. Heart failure according to the present invention includes overt and/or advanced heart failure. In overt heart failure, the subject shows symptoms of heart failure as known to the person skilled in the art. Thus, it is in particular envisaged that the subject does not suffer from overt and/or advanced heart failure. Heart failure stages C and D of the ACC/AHA classification are considered as advanced heart failure.

The term "heart failure" as used herein in particular refers to stages C and D of the ACC/AHA classification and more preferably to stages A, B, C and D of the ACC/AHA classification.

Thus, in a preferred embodiment, the subject to be tested shall not suffer from heart failure classified as stage C or D according the ACC/AHA classification. Thus, it is envisaged that i) the subject does not suffer heart failure at all or ii) that the subject suffers from heart failure classified as stage A or B according the ACC/AHA classification.

In another preferred embodiment, the subject to be tested does not suffer from heart failure classified as stage A, B, C or D according the ACC/AHA classification. Thus, the subject shall not suffer from heart failure at all.

The ACC/AHA classification is a classification for heart failure developed by the American College of Cardiology and the American Heart Association (for the classification, see J. Am. Coll. Cardiol. 2001;38;2101-2113, updated in 2005, see J. Am. Coll. Cardiol. 2005;46;e1-e82 which is herewith incorporated by reference in its entirety). The ACC/AHA classification is also referred to as ACCF/AHA classification. ACC: American College of Cardiology, ACCF: American College of Cardiology Foundation, AHA: American Heart Association.

The classification is also described in Mureddu et al., European Journal of Heart Failure (2012) 14, 718-729 which is herewith incorporated by reference as well. Four stages A, B, C and D are defined. Stages A and B are not HF (heart failure) but are considered to help identify patients early before developing "truly" HF. Stages A and B patients are best defined as those with risk factors for the development of HF. For example, patients with coronary artery disease, hypertension, or diabetes mellitus who do not yet demonstrate impaired left ventricular (LV) function, hypertrophy, or geometric chamber distortion would be considered stage A, whereas patients who are asymptomatic but demonstrate impaired LV function would be designated as stage B. Stage C then denotes patients with current or past symptoms of HF associated with underlying structural heart disease (the bulk of patients with HF), and stage D designates patients with truly refractory HF. The ACC/AHA classification is also explained in WO 2012/025355 which herewith is incorporated by reference in its entirety.

Stages A to D of heart failure according to the ACCF/AHA classification are also described in Jessup et al. (Circulation. 2013;128:e240-e327) which herewith is incorporated by reference as well.

The definition of the various stages is as preferably as follows. The following includes a description of the stage and examples for pathophysiological states in individuals which would be classified in the respective stage.

### Stage A:

Description: Patients at high risk of developing HF because of the presence of conditions that are strongly associated with the development of HF. Such patients have no identified structural or functional abnormalities of the pericardium, myocardium, coronary circulation or cardiac valves and have never shown signs or symptoms of HF.

Examples: Systemic hypertension; coronary artery disease; diabetes mellitus; history of cardiotoxic drug therapy or alcohol abuse; personal history of rheumatic fever; family history of cardiomyopathy.

### Stage B:

Description: Patients who have developed structural heart disease that is strongly associated with the development of HF but who have never shown signs or symptoms of HF.

Examples: Left ventricular hypertrophy or fibrosis; left ventricular dilatation or hypo contractility; asymptomatic valvular heart disease; previous myocardial infarction.

### Stage C:

Description: Patients who have current or prior symptoms of HF associated with underlying structural heart disease.

Examples: Dyspnea or fatigue due to left ventricular systolic dysfunction; asymptomatic patients who are undergoing treatment for prior symptoms of HF.

### Stage D:

Description: Patients with advanced structural heart disease and marked symptoms of HF at rest despite maximal medical therapy and who require specialized interventions.

Examples: Patients who are frequently hospitalized for HF or cannot be safely discharged from the hospital; patients in the hospital awaiting heart transplantation; patients at home receiving continuous intravenous support for symptom relief or being supported with a mechanical circulatory assist device; patients in a hospice setting for the management of HF.

Further, it is envisaged that the subject to be tested does not suffer from diabetes mellitus, the metabolic syndrome, and/or impaired renal function.

The subject to be tested does not suffer from hypertension. The term "hypertension" is well known in the art. Preferably, it is a long term medical condition in which the blood pressure in the arteries is persistently elevated. Accordingly, the blood pressure shall be higher than normal. Normal blood pressure is, preferably within the range of 100-140 millimeters mercury (mmHg) systolic and 60-90 mmHg diastolic. Accordingly, a subject who suffers from hypertension preferably has a systolic blood pressure of equal to or greater than 140 mm Hg and/or a diastolic blood pressure of equal to or greater than to 90 mm Hg. Accordingly, a subject who does not suffer from hypertension preferably has a systolic blood pressure of lower than 140 mm Hg and/or a diastolic blood pressure of lower than to 90 mm Hg
Preferably, the subject to be tested is not an athlete.

Further, it is envisaged that the subject is not pregnant.

The term "sample" refers to a sample of a blood, serum or plasma sample. Samples of body fluids can be obtained by well-known techniques and include, samples of blood, plasma, serum, urine, lymphatic fluid, sputum, ascites, or any other bodily secretion or derivative thereof. Tissue or organ samples may be obtained from any tissue or organ by, e.g., biopsy. Separated cells may be obtained from the body fluids or the tissues or organs by separating techniques such as centrifugation or cell sorting. E.g., cell-, tissue- or organ samples may be obtained from those cells, tissues or organs which express or produce the biomarker. The sample may be frozen, fresh, fixed (e.g. formalin fixed), centrifuged, and/or embedded (e.g. paraffin embedded), etc. The cell sample can, of course, be subjected to a variety of well-known post-collection preparative and storage techniques (e.g., nucleic acid and/or protein extraction, fixation, storage, freezing, ultrafiltration, concentration, evaporation, centrifugation, etc.) prior to assessing the amount of the marker in the sample.

The sample is a blood, serum or plasma sample. Thus, it is envisaged that the sample is whole blood, a blood serum or blood plasma sample.

Serum is the liquid fraction of whole blood that is obtained after the blood is allowed to clot. For obtaining the serum, the clot is removed by centrifugation and the supernatant is collected. Plasma is the acellular fluid portion of blood. For obtaining a plasma sample, whole blood is collected in anticoagulant-treated tubes (e.g. citrate-treated or EDTA-treated tubes). Cells are removed from the sample by centrifugation and the supernatant (i.e. the plasma sample) is obtained.

The term "measuring" the amount of a biomarker as referred to herein refers to the quantification of the biomarker, e.g. to determining the level of the biomarker in the sample, employing appropriate methods of detection described elsewhere herein. The terms "measuring" and "determining" are used herein interchangeably.

In an embodiment, the amount of a biomarker is measured by contacting the sample with an agent that specifically binds to the biomarker, thereby forming a complex between the agent and said biomarker, detecting the amount of complex formed, and thereby measuring the amount of said biomarker.

The biomarkers as referred to herein can be detected using methods generally known in the art. Methods of detection generally encompass methods to quantify the amount of a biomarker in the sample (quantitative method). It is generally known to the skilled artisan which of the following methods are suitable for qualitative and/or for quantitative detection of a biomarker. Samples can be conveniently assayed for, e.g., proteins using Westerns and immunoassays, like ELISAs, RIAs, fluorescence-based immunoassays, which are commercially available. Further suitable methods to detect biomarker include measuring a physical or chemical property specific for the peptide or polypeptide such as its precise molecular mass or NMR spectrum. Said methods comprise, e.g., biosensors, optical devices coupled to immunoassays, biochips, analytical devices such as mass- spectrometers, NMR- analyzers, or chromatography devices. Further, methods include microplate ELISA-based methods, fully-automated or robotic immunoassays (available for example on El-ecsys^{™} analyzers), CBA (an enzymatic Cobalt Binding Assay, available for example on Roche-Hitachi^{™} analyzers), and latex agglutination assays (available for example on Roche-Hitachi^{™} analyzers).

For the detection of biomarker proteins as referred to herein a wide range of immunoassay techniques using such an assay format are available, see, e.g., U.S. Pat. Nos. 4,016,043, 4,424,279, and 4,018,653. These include both single-site and two-site or "sandwich" assays of the non-competitive types, as well as the traditional competitive binding assays. These assays also include direct binding of a labeled antibody to a target biomarker.

Sandwich assays are among the most useful immunoassays.

Methods for measuring electrochemiluminescent phenomena are well-known. Such methods make use of the ability of special metal complexes to achieve, by means of oxidation, an excited state from which they decay to ground state, emitting electrochemiluminescence. For review see Richter, M.M., Chem. Rev. 104 (2004) 3003-3036.

In an embodiment, the antibody (or antigen-binding fragment thereof) to be used for measuring the amount of a biomarker is ruthenylated. Accordingly, the antibody (or antigen-binding fragment thereof) shall comprise a ruthenium label. In an embodiment, said ruthenium label is a bipyridine-ruthenium(II) complex.

Measuring the amount of a polypeptide (such as soluble ST2) may, preferably, comprise the steps of (a) contacting the polypeptide with an agent that specifically binds said polypeptide (b) (optionally) removing non-bound agent, (c) measuring the amount of bound binding agent, i.e. the complex of the agent formed in step (a). According to a preferred embodiment, said steps of contacting, removing and measuring may be performed by an analyzer unit. According to some embodiments, said steps may be performed by a single analyzer unit of said system or by more than one analyzer unit in operable communication with each other. For example, according to a specific embodiment, said system disclosed herein may include a first analyzer unit for performing said steps of contacting and removing and a second analyzer unit, operably connected to said first analyzer unit by a transport unit (for example, a robotic arm), which performs said step of measuring.

The bound agent, i.e. the agent or the agent/polypeptide complex, will generate an intensity signal. Binding according to the present invention includes both covalent and non-covalent binding.

The agent which specifically binds the biomarker (herein also referred to as "binding agent") may be coupled covalently or non-covalently to a label allowing detection and measurement of the bound agent. Labeling may be done by direct or indirect methods. Direct labeling involves coupling of the label directly (covalently or non-covalently) to the binding agent. Indirect labeling involves binding (covalently or non-covalently) of a secondary binding agent to the first binding agent. The secondary binding agent should specifically bind to the first binding agent. Said secondary binding agent may be coupled with a suitable label and/or be the target (receptor) of tertiary binding agent binding to the secondary binding agent. Suitable secondary and higher order binding agents may include antibodies, secondary antibodies, and the well-known streptavidin-biotin system (Vector Laboratories, Inc.). The binding agent or substrate may also be "tagged" with one or more tags as known in the art. Such tags may then be targets for higher order binding agents. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star^{™} (Amersham Bio-sciences), ECF^{™} (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. A radioactive label can be detected by any method known and appropriate, e.g. a light-sensitive film or a phosphor imager.

The amount of a polypeptide may be, also preferably, measured as follows: (a) contacting a solid support comprising a binding agent for the polypeptide as described elsewhere herein with a sample comprising the peptide or polypeptide and (b) measuring the amount of peptide or polypeptide which is bound to the support. Materials for manufacturing supports are well-known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc.

In yet an aspect the sample is removed from the complex formed between the binding agent and the at least one marker prior to the measurement of the amount of formed complex. Accordingly, in an aspect, the binding agent may be immobilized on a solid support. In yet an aspect, the sample can be removed from the formed complex on the solid support by applying a washing solution. The formed complex shall be proportional to the amount of the at least one marker present in the sample. It will be understood that the specificity and/or sensitivity of the binding agent to be applied defines the degree of proportion of at least one marker comprised in the sample which is capable of being specifically bound. Further details on how the measurement can be carried out are also found elsewhere herein. The amount of formed complex shall be transformed into an amount of at least one marker reflecting the amount indeed present in the sample. Such an amount, in an aspect, may be essentially the amount present in the sample or may be, in another aspect, an amount which is a certain proportion thereof due to the relationship between the formed complex and the amount present in the original sample.

The terms "binding agent", "detection agent" and "agent that specifically binds to a biomarker" are used interchangeably herein. Preferably to an agent that comprises a binding moiety which specifically binds the corresponding to the respective biomarker. Examples of "binding agents" or "agents" are a nucleic acid probe, nucleic acid primer, DNA molecule, RNA molecule, aptamer, antibody, antibody fragment, peptide, peptide nucleic acid (PNA) or chemical compound. A preferred agent is an antibody which specifically binds to the biomarker to be measured. The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity (i.e. antigen-binding fragments thereof). Preferably, the antibody is a polyclonal antibody. More preferably, the antibody is a monoclonal antibody.

The term "specific binding" or "specifically bind" refers to a binding reaction wherein binding pair molecules exhibit a binding to each other under conditions where they do not significantly bind to other molecules. The term "specific binding" or "specifically binds", when referring to a protein or peptide as biomarker, refers to a binding reaction wherein a binding agent binds to the corresponding biomarker with an affinity of at least 10⁻⁷ M. The term "specific binding" or "specifically binds" preferably refers to an affinity of at least 10⁻⁸ M or even more preferred of at least 10⁻⁹ M for its target molecule. The term "specific" or "specifically" is used to indicate that other molecules present in the sample do not significantly bind to the binding agent specific for the target molecule.

ST2, also known as "Interleukin 1 receptor-like 1" is a member of the IL-1 receptor family that is produced by cardiac fibroblasts and cardiomyocytes under conditions of mechanical stress. ST2 is an interleukin-1 receptor family member and exists in a membrane-bound isoform and a soluble isoform (sST2). In the context of the present invention, the amount of soluble ST2 shall be measured (see Dieplinger et al. (Clinical Biochemistry, 43, 2010: 1169 to 1170). ST2 also known as Interleukin 1 receptor-like 1 or IL1RL1, is encoded in humans by the IL1RL1 gene. The sequence of the human ST2 polypeptide is well known in the art, and e.g. accessible via GenBank, see NP_003847.2 GI:27894328.

The term "amount" as used herein encompasses the absolute amount of a biomarker as referred to herein, the relative amount or concentration of the said biomarker as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response amounts measured from biological read out systems in response to the peptides or intensity signals obtained from specifically bound ligands. It is to be understood that values correlating to the aforementioned amounts or parameters can also be obtained by all standard mathematical operations.

The term "comparing" as used herein refers to comparing the amount of the biomarker in the sample from the subject with the reference amount of the biomarker specified elsewhere in this description. It is to be understood that comparing as used herein usually refers to a comparison of corresponding parameters or values, e.g., an absolute amount is compared to an absolute reference amount while a concentration is compared to a reference concentration or an intensity signal obtained from the biomarker in a sample is compared to the same type of intensity signal obtained from a reference sample. The comparison may be carried out manually or computer-assisted. Thus, the comparison may be carried out by a computing device. The value of the measured or detected amount of the biomarker in the sample from the subject and the reference amount can be, e.g., compared to each other and the said comparison can be automatically carried out by a computer program executing an algorithm for the comparison. The computer program carrying out the said evaluation will provide the desired assessment in a suitable output format. For a computer-assisted comparison, the value of the measured amount may be compared to values corresponding to suitable references which are stored in a database by a computer program. The computer program may further evaluate the result of the comparison, i.e. automatically provide the desired assessment in a suitable output format. For a computer-assisted comparison, the value of the measured amount may be compared to values corresponding to suitable references which are stored in a database by a computer program. The computer program may further evaluate the result of the comparison, i.e. automatically provides the desired assessment in a suitable output format.

In accordance with the present invention the amount of the biomarker soluble ST2 (or the amount of a BNP-type peptide or cardiac Troponin) shall be compared to a reference. The reference is preferably a reference amount. The term "reference amount" as used herein refers to an amount which allows for allocation of a subject into either (i) the group of being at risk of progressing to LVH or (ii) not being at risk of progressing to LVH. A suitable reference amount may be determined from a reference sample to be analyzed together, i.e. simultaneously or subsequently, with the test sample.

Reference amounts can, in principle, be calculated for a cohort of subjects as specified above based on the average or mean values for a given biomarker by applying standard methods of statistics. In particular, accuracy of a test such as a method aiming to identifying a subject is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The clinical performance of a method depends on its accuracy, i.e. its ability to correctly allocate subjects to a certain prognosis or diagnosis. The ROC plot indicates the overlap between the two distributions by plotting the sensitivity versus 1 -specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1 -specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/- specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis for a given event with a proper balance of sensitivity and specificity, respectively. Accordingly, the reference to be used for the aforementioned method of the present invention, i.e. a threshold which allows to differentiating between subjects being at risk of progressing to LVH or those not being at risk of progressing to LVH among a cohort of subjects (as described elsewhere herein) can be generated, preferably, by establishing a ROC for said cohort as described above and deriving a threshold amount therefrom. Dependent on a desired sensitivity and specificity for the diagnostic method, the ROC plot allows deriving suitable thresholds. It will be understood that an optimal sensitivity is desired for excluding a subject being at risk of progressing to LVH (i.e. a rule out) whereas an optimal specificity is envisaged for a subject to be assessed as being at risk of progressing to LVH (i.e. a rule in).

It is envisaged that the reference amount is an age-specific reference amount. This applies in particular for BNP-type peptides. As set forth elsewhere herein the methods of the present invention preferably comprise the measurement of the amount of a BNP-type peptide such as BNP or NT-proBNP in a sample from the subject and the comparison to a reference amount. It is well known in the art that median of the amount of BNP-type peptide in the general population increases with the age, the reference amount for the BNP-type is preferably age-specific, i.e. depends on the age of the subject to be tested.

In certain embodiments, the term "reference amount" herein refers to a predetermined value. Said predetermined value shall allow for differentiating between subjects being at risk of progressing to LVH or those not being at risk of progressing to LVH.

Preferably, an amount of soluble ST2 in the sample of the test subject above the reference amount indicates that the subject is at risk of progressing to LVH. Also preferably, an amount of soluble ST2 in the sample below the reference amount indicates that the subject is not at risk of progressing to LVH.

Thus, the method of the present invention allows for the identification of a subject who is at risk of progression to LVH or for the identification of a subject who is not at risk of progressing to LVH.

In an embodiment, the reference amount is derived i) from a subject or a group thereof known not to be at risk of progressing to LVH, or ii) from a subject or a group thereof known to be at risk of progressing to LVH. Preferably, the reference amount is derived from a subject as defined herein above in connection with the term "subject" or group thereof.

If i) the reference amount is derived from a subject or a group thereof known not to be at risk of progressing to LVH, an amount of the biomarker soluble ST2 in the sample of the subject which is essentially the same as the reference amount or which is decreased as compared to the reference amount, is indicative for a subject who is not at risk of progressing to LVH.

If ii) the reference amount is derived from a subject or a group thereof known to be at risk of progressing to LVH, an amount of the biomarker soluble ST2 in the sample of the subject which is essentially the same as the reference amount or which is increased as compared to the reference amount, is indicative for a subject who is at risk of progressing to LVH.

The method of the present invention may further comprises the assessment of at least one further parameter in the subject to be tested. Preferably, said at least one further parameter is selected from the
- eGFR,
- the amount of a NT-proBNP or BNP in a sample from the subject, and
- the amount of a cardiac Troponin in a sample from the subject.

In a preferred embodiment, the following parameters are assessed: the amount of NT-proBNP or BNP in a sample from the subject and/or the amount of a cardiac Troponin in a sample from the subject. Thus, the amount of NT-proBNP or BNP and/or the amount of a cardiac Troponin is (are) measured. Subsequently, the amount(s) can be compared to a reference amount (or reference amounts).

In another preferred embodiment, the following parameters are assessed: the eGFR and the amount of NT-proBNP or BNP in a sample from the subject.

In another preferred embodiment, the method of the present invention comprises the assessment of all parameters as referred above: the eGFR, the amount of NT-proBNP or BNP

in a sample from the subject, and the amount of a cardiac Troponin in a sample from the subject.

### Parameters "BNP-type peptide" and "cardiac Troponin"

In a preferred embodiment, the method may further comprise the measurement of the amount at least one further biomarker, i.e. of NT-proBNP or BNP and/or a of a cardiac Troponin. Preferably, the method further comprises the measurement of the amount of NT-proBNP. Also preferably, the method further comprises the measurement of the amount of a cardiac Troponin. Moreover, it is envisaged that the method further comprises the measurement of the amounts of a cardiac Troponin and NT-proBNP or BNP. In a further step, the amount (or amounts) is (are) compared with a reference. In an embodiment, said reference is a reference amount as specified elsewhere herein.

The term "cardiac Troponin" refers to all Troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, the term "cardiac Troponin" is Troponin I, and, more preferably, to Troponin T.

The Brain Natriuretic Peptide type peptide (herein also referred to as BNP-type peptide) is selected from the group consisting of NT-proBNP and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP). Preferably, brain natriuretic peptides according to the present invention are NT-proBNP, BNP, and variants thereof. BNP is the active hormone and has a shorter half-life than its respective inactive counterpart NT-proBNP. Preferably, the Brain Natriuretic Peptide-type peptide is BNP (Brain natriuretic peptide), and more preferably NT-proBNP (N-terminal of the prohormone brain natriuretic peptide).

The additional measurement of the amount of NT-proBNP or BNP is in particular advantageous in subjects having an increased level of sST2 (i.e. in subjects in which the amount of sST2 in the sample is above the reference amount). As set forth above, these subjects are at risk of progressing to LVH. The additional determination of the amount of NT-proBNP or BNP in these subjects (i.e. in a sample from these subjects) allows for differentiating whether the subject is at short term risk or at long term risk of progressing to LVH. Subjects having increased levels of sST2 and a BNP-type peptide are in the transition to LVH. Thus, an amount of NT-proBNP or BNP in a (the) sample from the subject which is above the reference amount (for the NT-proBNP or BNP) is preferably indicative for a subject who is at short term risk of progressing to LVH. An amount of NT-proBNP or BNP in a (the) sample from the subject which is below the reference amount (for the NT-proBNP or BNP) is preferably indicative for a subject who is at long term risk of progressing to LVH. Thus, an amount of NT-proBNP or BNP in the sample from the subject which is below the reference amount is indicative for a subject who is not at short term risk of progressing to LVH.

Thus, it is in particular contemplated that the methods of the present invention comprises the measurement of sST2 and NT-proBNP or BNP and the comparison of the amount of sST2 to a reference amount (for sST2) and the amount of the NT-proBNP or BNP to a reference amount (for NT-proBNP or BNP). Preferably, an amount of sST2 above the reference and (i.e. in combination with) an amount of NT-proBNP or BNP above the reference is indicative for a subject who is at short term risk of progressing to LVH and/or wherein an amount of sST2 above the reference and (i.e. in combination with) an amount of NT-proBNP or BNP below the reference is indicative for a subject who is not at short term risk of progressing to LVH. However, the subject who is a short term risk of progression to LVH is at long term risk of progression to LVH.

A subject who is at short term risk of progressing to LVH is considered as a subject who is at risk of progressing to LVH within a short interval. (in particular after the sample has been obtained). Such a subject is at immediate risk of progressing to LVH. A subject who is at long term risk of progressing to LVH is considered as a subject who is at risk of progressing to LVH within a long interval (in particular after the sample has been obtained). Preferably, the subject who is at long term risk is not at short term risk. Thus, the subject is not at immediate risk of progressing to LVH and consequently is likely not to progress to LVH within a short interval (after the sample has been obtained).

A short interval (or short term) in the sense of the present invention is preferably an interval of about one month to about three years, more preferably an interval of about one month to about two years, in particular of about one month to about 18 months (preferably after the sample has been obtained).

A long interval (long term) in the sense of the present invention is preferably an interval of about three years to about 20 years, more preferably of about five years to about 20 years, most preferably an interval of about five years to about 17 years.

Further long interval (long term) in the sense of the present invention can be an interval of about five years to about 15 years, or about seven to about 12 years.

The definitions given herein above apply mutatis mutandis to the following methods and uses of the present invention.

As set forth above, the measurement of a BNP-type peptide allows assessing whether a subject who has an increased sST2 is at long term risk or short term risk of progressing to LVH.

Accordingly, a non-claimed disclosure relates to a method for differentiating in a subject who has an increased level of sST2, preferably in a blood, serum or plasma sample, between a short term risk of progressing to LVH and a long term risk of progressing to LVH (i.e. not at short term risk), said method comprising the steps of
(a) measuring the amount of a BNP-type peptide such as NT-proBNP or BNP in a sample from the subject, and
(b) comparing the thus measured amount to a reference amount, and optionally
(c) differentiating between a short term risk of progressing to LVH and at long term risk of progressing to LVH.

Preferably, an amount of BNP-type peptide in the sample from the subject which is above the reference amount is indicative for a subject who is at short term risk of progressing to LVH. Preferably, an amount of BNP-type peptide in the sample from the subject which is below the reference amount is indicative for a subject who is not at short term risk of progressing to LVH. A subject who is not at short term risk of progressing to LVH however is at long term risk of progressing to LVH.

In an embodiment, a subject who has an increased level of sST2 displays a level, i.e. an amount, of sST2 in a sample such as a blood, serum or plasma sample above the reference amount. In a preferred embodiment, the level of sST2 is larger than 90% percentile for the biomarker in a cohort of subject such as in a cohort of subjects of the general population. Preferably, the subjects comprised the cohort have about the same age as the subject to be tested.

Further, the present disclosure relates to a method for identifying a subject who is at long term risk of progressing to LVH, said method comprising the steps of
(a) measuring the amount of a BNP-type peptide such as NT-proBNP or BNP and the amount of sST2 in a sample from the subject, and
(b) comparing the thus measured amounts to reference amounts, and optionally
(c) identifying a subject who is at long term risk of progressing to LVH.

Moreover, the present disclosure relates to a method for predicting whether a subject is at long term risk of progressing to LVH, said method comprising the steps of
(a) measuring the amount of a BNP-type peptide such as NT-proBNP or BNP and the amount of sST2 in a sample from the subject, and
(b) comparing the thus measured amounts to reference amounts, and optionally
(c) predicting whether the subject is at long term risk of progressing to LVH.

Preferably, an amount of sST2 above the reference amount in combination with an amount of a BNP-type peptide below the reference amount is indicative for a subject who is at long term risk of progressing to LVH.

Moreover, the present disclosure relates to a method for identifying a subject who is at short term risk of progressing to LVH, said method comprising the steps of
(a) measuring the amount of a BNP-type peptide such as NT-proBNP or BNP and the amount of sST2 in a sample from the subject, and
(b) comparing the thus measured amounts to reference amounts, and optionally
(c) identifying a subject who is at short term risk of progressing to LVH.

Moreover, the present disclosure relates to a method for predicting whether (or not) a subject is at short term risk of progressing to LVH, said method comprising the steps of
(a) measuring the amount of a BNP-type peptide such as NT-proBNP or BNP and the amount of sST2 in a sample from the subject, and
(b) comparing the thus measured amounts to reference amounts, and optionally
(c) predicting whether the subject is at short term risk of progressing to LVH (or not).

Preferably, an amount of sST2 above the reference amount in combination with an amount of a BNP-type peptide above the reference amount is indicative for a subject who is at short term risk of progressing to LVH. Moreover, an amount of sST2 above the reference amount in combination with an amount of a BNP-type peptide below the reference amount is indicative for a subject who is not at short term risk of progressing to LVH (and thus for a subject who is at long term risk). Moreover, an amount of sST2 below the reference amount in combination with an amount of a BNP-type peptide below the reference amount is indicative for a subject who is not at short term risk of progressing to LVH.

In step (b) of the aforementioned methods it is envisaged that the amount of the BNP-type peptide is compared to a reference amount for the BNP-type peptide and the amount of sST2 is compared to a reference amount for sST2.

### Parameter "eGFR"

The parameter eGRF is preferably assessed by determining the eGFR (estimated glomerular filtration rate) in a sample from the subject. The eGFR is the estimated Glomerular Filtration Rate. The term is well known in the art and e.g. described in Chowdhury which herewith is incorporated by reference (American Journal of Hypertension, 2015, 28(3): 380ff). It is an estimation of the flow rate of filtered fluid through the kidney, i.e. an estimation of the volume of fluid filtered from the renal (kidney) glomerular capillaries into the Bowman's capsule per unit time. The eGFR is based on the amount of Creatinine in a serum sample of the subject. A number of formulae have been devised to estimate GFR values on the basis of serum creatinine levels.

### Parameter "blood pressure"

The method of the present invention may also comprise the assessment of the blood pressure of the subject, wherein the assessment of the blood pressure is not practiced on the human or animal body. The blood pressure can be the systolic or diastolic blood pressure. In particular, the term "blood pressure" refers to the systolic blood pressure. Thus, the method may comprise the provision of a blood pressure value, e.g. it is envisaged to assess the blood pressure from the medical records of the subject. Thus, the method of the present invention may comprise the step of providing a blood pressure value of the subject based on the medical records of the subject.

In an embodiment of the method of the present invention, the at least one further parameter is compared to a suitable reference. Thus, it is contemplated to compare the amount of BNP-type peptide to a reference amount (for the BNP-type peptide), to compared the amount of the cardiac Troponin to a reference amount (for the cardiac Troponin), to compared the eGFR value to a reference eGFR value, and/or the blood pressure value to a reference blood pressure value. Based on the results of the comparisons made in accordance with the present invention, the subject is identified.

Said reference amounts or reference values can be obtained as described above in connection with the reference amount for soluble ST2, e.g. from a subject or group of subjects as described above (such as a subject known to be at risk of progressing to LVH or a subject known to be not at risk of progressing to LVH).

In the following the kind of direction of change for the additional parameter is indicated, and thus whether an amount or value above or below the reference is indicative for a subject who is at risk (or not at risk).

Preferably, an amount of a cardiac Troponin and/or BNP-type peptide in the sample of the test subject above the reference amount indicates that the subject is at risk of progressing to LVH. Also preferably, an amount of cardiac Troponin and/or a BNP-type peptide in the sample below the reference amount indicates that the subject is not at risk of progressing to LVH.

Preferably, an eGFR value in the test subject below the reference eGFR value indicates that the subject is at risk of progressing to LVH. Also preferably, an eGFR value in the test subject above the reference eGFR value indicates that the subject is not at risk of progressing to LVH.

The parameter(s) shall be assessed in addition to the amount of sST2. Thus, the diagnostic algorithm is evident from the kind of direction for the parameters given above. E.g., an amount of sST2 above the reference amount in combination with an amount of cardiac Troponin and/or BNP-type peptide in the sample of the test subject above the reference amount indicates that the subject is at risk of progressing to LVH.

In an embodiment, a score is calculated based on the amount of sST2 and on the value(s) for the least one further parameter (such as the amount of a BNP-type peptide) and the comparison to a suitable reference score.

In an embodiment of the method of the invention, said method further comprises a step of recommending and/or initiating at least one patient management measure according to the comparison results. Preferably, said at least one patient management measure is recommended or initiated if the subject is at risk of progressing to left ventricular hypertrophy.

The term "recommending" as used herein means establishing a proposal for a suitable supportive measure which could be applied to the subject. The patient management measure refers to all measures which can be applied to subjects being at risk of progressing to LVH in order to avoid or delay the progression to LVH. For example, patient management measures include degree of monitoring (e.g., close, regular or weak monitoring), drug treatment, or life style recommendations. In particular, said at least one patient management measure is selected from the group consisting of: close monitoring, administration of antihypertensive medication, and life style changes. In an embodiment, drug treatment such as administration is of antihypertensive medication is recommended (or initiated), if the subject is at short term risk of progressing to LVH.

A preferred antihypertensive medication is selected from the group consisting of angiotensin-converting enzyme (ACE) inhibitors, Angiotensin-II-receptor blocker (ARB), aldosteron antagonists, and beta blockers.

The beta blocker is preferably selected from proprenolol, metoprolol, bisoprolol, carvedilol, bucindolol and nebivolol.

The ACE inhibitor is preferably selected from Enalapril, Captopril, Ramipril and Trandolapril.

The aldosterone antagonist is preferably selected from Eplerone, Spironolactone, Canrenone, Mexrenone and Prorenone.

Life style changes to be recommended or initiated are preferably selected from smoking cessation, moderation of alcohol consumption, increased physical activity, weight loss and sodium (salt) restriction.

The definitions given herein above apply mutatis mutandis to the following.

Accordingly, the present invention relates to a method for predicting the risk of a subject of progressing to LVH according to claim 7; further disclosure relates to a method for predicting the risk of a subject of progressing to LVH, said method comprising:
(a) measuring the amount of the biomarker soluble ST2 in a sample from the subject, and
(b)comparing the amount of soluble ST2 to a reference.

Preferably, the risk is predicted by carrying out the further step (c) of predicting the risk, or of providing a prediction of the risk of the subject of progressing to LVH. Said step is based on the results of step (b).

In accordance with the present invention, the risk of a subject of progressing to LVH shall be predicted. Thus, a subject can be identified who is at risk of progressing to LVH or who is not at risk thereof. The term "predicting the risk" as used herein, preferably, refers to assessing the probability according to which a subject will progress to LVH. More preferably, the risk/probability within a certain time window is predicted. Preferred predictive windows are given herein above in connection with the method of identifying a subject who is at risk of progressing to LVH. In a preferred embodiment of the present invention, the predictive window, preferably, is a period of at least about 5 years, or in particular of at least about 10 years. In a particular preferred embodiment of the present invention, the predictive window, preferably, is a period of about 17 years (or more). Moreover, the predictive window can be a period of about 20 years. Preferably, said predictive window is calculated from the time point at which the sample to be tested has been obtained.

Thus, it is in particular envisaged by the present invention to predict the risk of progressing to LVH within about 5 years, about 10 years, about 17 years or about 20 years.

Further, it is envisaged to the predict whether subject is at short term risk (or not), or whether a subject is at short term or long term risk of progressing to LVH. Preferred intervals for the long term and short term prediction are disclosed above in connecting with the method of identifying a subject who is at risk or progressing to LVH. Preferred diagnostic algorithms are also disclosed above.

As will be understood by those skilled in the art, such a prediction is usually not intended to be correct for 100% of the subjects. The term, however, requires that prediction can be made for a statistically significant portion of subjects in a proper and correct manner. Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98%, or at least 99%. The p-values are, preferably, 0.1, 0.05, 0.01, 0.005, or 0.0001. Preferably, the probability envisaged by the present invention allows that the prediction of an increased, normal or decreased risk will be correct for at least 60%, at least 70%, at least 80%, or at least 90% of the subjects of a given cohort or population. The term, preferably, relates to predicting whether a subject is at elevated risk or reduced risk as compared to the average risk in a population of subjects. The terms "elevated risk" and "reduced risk" have been defined in connection with the method of identifying a subject who is at risk.

The term "predicting the risk" as used herein means that the subject to be analyzed by the method of the present invention is allocated either into the group of subjects being at risk of progressing to LVH, or into the group of subjects being not at risk of progressing to LVH.

The terms "sample", "subject", "LVH", and "reference amount" have been defined above. In addition, preferred diagnostic algorithms are indicated above. The definitions and explanations apply accordingly to the method of risk prediction.

The method of the present invention may further comprise the assessment of at least one further parameter in the subject to be tested as described above. Preferably, said at least one further parameter is selected from the eGFR, the amount of NT-proBNP or BNP in a sample from the subject, and the amount of a cardiac Troponin in a sample from the subject. Preferred combinations are described herein above.

In an embodiment of the method of the present invention, the at least one further parameter is compared to a suitable reference. Thus, it is contemplated to compare the amount of NT-proBNP or BNP to a reference amount (for the NT-proBNP or BNPe), to compare the amount of the cardiac Troponin to a reference amount (for the cardiac Troponin), and/or to compare the eGFR value to a reference eGFR value. Based on the results of the comparisons made in accordance with the present invention, the risk is predicted.

In addition, it is contemplated that the short term risk or the long term risk of progressing to LVH is predicted. In an embodiment, it predicted whether the subject is at short term risk or progression to LVH (or not). In another embodiment, it is predicted whether a subject is a long term risk (or not). This prediction is preferably based on the measurement of the amount of a BNP-type peptide and the amount of sST2 and the comparison of the amount of a BNP-type peptide to a reference amount (for the BNP-type peptide) and the comparison of the amount of sST2 to a reference amount (for sST2). Preferred diagnostic algorithms are disclosed herein elsewhere.

In an embodiment of the method of the invention, said method further comprises a step of recommending and/or initiating at least one patient management measure according to the comparison results. Preferably, said at least one patient management measure is recommended or initiated if the subject is at risk of progressing to left ventricular hypertrophy. Preferred patient management measures are described above.

The present invention further relates to the *in vitro* use of sST2 or of an agent that specifically binds to sST2 according to claim 9; further disclosure relates to the use of sST2 or of an agent that specifically binds to sST2 in a sample of a subject (as defined above) for identifying a subject who is at risk of progressing to left ventricular hypertrophy (LVH) or for predicting the risk of a subject of progressing to LVH.

The term "agent that specifically binds to a biomarker" are used interchangeably herein. Preferably it relates to an agent that comprises a binding moiety which specifically binds the corresponding biomarker. Examples of "binding agents" or "agents" are a nucleic acid probe, nucleic acid primer, DNA molecule, RNA molecule, aptamer, antibody, antibody fragment, peptide, peptide nucleic acid (PNA) or chemical compound. A preferred agent is an antibody which specifically binds to the biomarker to be measured. The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity (i.e. antigen-binding fragments thereof). Preferably, the antibody is a polyclonal antibody. More preferably, the antibody is a monoclonal antibody.

The aforementioned uses may further encompass the use of NT-proBNP or BNP (or of an agent that specifically binds thereto), a cardiac Troponin (or an agent that specifically binds thereto) in the sample of the subject. Further, it may encompass the use of means for determining the eGFR.

In a preferred embodiment the aforementioned used further encompass the of NT-proBNP or BNP or of an agent that specifically binds thereto. The additional use of NT-proBNP or BNP or of an agent that specifically binds thereto allows differentiating between a long term risk and a short term risk in a subject.

Accordingly, the present disclosure relates to the use of
(i) sST2 (soluble ST2) and a NT-proBNP or BNP, and/or
(ii) an agent which specifically binds to sST2 and an agent which specifically binds to NT-proBNP or BNP
in a sample from a subject as defined herein for a) identifying a subject who is at long term risk of progressing to LVH, for predicting whether a subject is at long term risk of progressing to LVH, for identifying a subject who is at short term risk of progressing to LVH, or for predicting whether a subject is at short term risk of progressing to LVH.

Finally, the present disclosure relates to the use of BNP or NT-proBNP in a sample from a subject who has an increased level of sST2 (preferably in a blood, serum or plasma sample) for differentiating in said subject between a short term risk of progressing to LVH and a long term risk of progressing to LVH. Also, the present disclosure relates to the use of BNP or NT-proBNP in a sample from a subject who has an increased level of sST2 (preferably in a blood, serum or plasma sample) for differentiating between a subject who is a short term risk of progressing to LVH and a subject who is not at short term risk of progressing to LVH.

### EXAMPLES

The following Examples shall illustrate the invention. They shall, however, not be construed as limiting the scope of the invention.

### EXAMPLE 1: Design of a LVH outcome case control study with samples from the Stanislas study

### Table 1: Characteristics of patients in the LVH case control study

Legend: The LVH outcome case control study was designed with samples from participants of the Stanislas cohort. Participants of the Stanislas cohort are families with two biological parents and at least two children (Siest et al., 2008). The Stanislas cohort has been followed with 3 health examinations and blood samplings at visit2 (V2) 5 years after, at visit3 (V3) 10 years after and visit4 (V4) 17 years after the initial examination, visit1 (V1).

The size of the LVH case control study comprised serial samples of 4 visits (V1-4) from 202 subjects, out of which 101 subjects progressed to LVH at V4. At the initial examination, at V1 absence of LVH was shown for all 202 subjects of the case control study. LVH outcome cases were matched to 101 controls for the presence or absence of LVH identified during the visit4 (V4). The matching included age, gender and systolic blood pressure at the first visit, as these variables are strongly associated with LV mass in the available literature. The outcome variable LV mass was calculated with the ASE method, indexed on body surface area (Dubois'formula), dichotomized (according to the cut-offs of Lang et al.2015). The matching process at V4 comprised: One patient without LVH (<96 g/m² in women and 116 g/m² in men) was matched with one patient with LVH (>=96g/m² in women and 116 g/m² in men) with the following prerequisites:
1/ Same gender
2/ Same age (1 year precision)
3/ Delta in indexed LV mass > 20 g/m2 to avoid the matching of patients with close LV mass
4/ Delta in systolic BP at first visit <=10 mmHg.

Patient characteristics were compared between LVH and no LVH patients at V4 and at baseline (V1).

### Characteristics of patients at Visit 1 (V1)

| **Parameter** | | **No LVH N=101** | **LVH N=101** | **p-value** | **No LVH N=101** | **LVH N=101** |
|---|---|---|---|---|---|---|
| **Demography** | | | | | **means** | |
| | Age (years) | 43.5 (41.3 - 46.2) | 43.6 (41.0 - 46.5) | 0.53 | 44.0 ± 3.3 | 44.0 ± 3.4 |
| | Female gender (%) | 45.5 | 45.5 | 1.00 | | |
| | Smoking habit (%) | 16.2% | 20.0% | 0.57 | | |

| **Haemodynamics** | | | | | | |
|---|---|---|---|---|---|---|
| | Office Sytolic BP (mmHg) | 123.4±9.9 | 123±10.6 | 0.50 | | |
| | Office Diastolic BP (mmHg) | 75.3±9.9 | 76.1±8.2 | 0.48 | | |
| | Heart rate (bpm) | 66.1 ± 9.9 | 66.5 ± 10.8 | 0.82 | | |

| **Medical history** | | | | | | |
|---|---|---|---|---|---|---|
| | Type II diabetes | 2.0% | 0% | NA | | |

| **Biology** | | | | | | |
|---|---|---|---|---|---|---|
| | Total cholesterol (g/L) | 5.79 ± 0.95 | 5.87 ± 0.99 | 0.56 | | |
| | HDL cholesterol (g/L) | 1.49 ± 0.38 | 1.40 ± 0.40 | 0.0693 | | |
| | LDL cholesterol (g/L) | 3.84 ± 0.94 | 3.94 ± 0.97 | 0.39 | | |
| | Triglycerids (g/L) | 0.82 (0.62 - 1.29) | 0.93 (0.69 - 1.44) | 0.31 | 1.01 ± 0.59 | 1.15 ± 0.73 |
| | eGFR MDRD (ml/min/1.73m2) | 83.8 ± 12.8 | 84.8 ± 11.8 | 0.56 | | |

| | | | | | | |
|---|---|---|---|---|---|---|
| Values are m±SD or mediane(IQR), % | | | | | | |

### Characteristics of patients at Visit 4 (V4)

| **Parameter** | | **No LVH N=101** | **LVH N=101** | **p-value** | **No LVH N=101** | **LVH N=101** |
|---|---|---|---|---|---|---|
| **Demography** | | | | | **means** | |
| | Age (years) | 61.0 (58.0 - 63.5) | 61.0 (58.0 - 63.0) | 1.00 | 61.2 ± 3.2 | 61.2±3.2 |
| | Female gender (%) | 45.5 | 45.5 | 1.00 | | |
| | Smoking habit (%) | 4.0% | 4.0% | 1.00 | | |

| **Haemodynamics** | | | | | | |
|---|---|---|---|---|---|---|
| | Office Systolic BP (mmHg) | 127.7 ± 14.3 | 130.8 ± 13.7 | 0.0824 | | |
| | Office Diastolic BP (mmHg) | 74.1 ± 8.1 | 75.1 ± 8.3 | 0.36 | | |
| | Heart rate (bpm) | 63.9 ± 9.0 | 63.1 ± 10.1 | 0.52 | | |

| **Medical history** | | | | | | |
|---|---|---|---|---|---|---|
| | Type II diabetes | | | | | |
| | LV mass (ASE, g) | 142.4±42.0 | 241.2±53.5 | <0.001 | | |
| | Indexed LV mass (ASE/Dubois, g/m2) | 77.6±17.8 | 128.7±22.2 | <0.001 | | |
| | History of Heart failure | 1.0% | 5.0% | 0.21 | | |
| | History of Arrhythmia | 16.2% | 12.9% | 0.55 | | |
| | History of myocardial infarction | 1.0% | 1.0% | 1 | | |
| | Prior stroke | 2.0% | 2.0% | 1 | | |
| | Hypertension | 29.3% | 38.6% | 0.18 | | |

| **Biology** | | | | | | |
|---|---|---|---|---|---|---|
| | Total cholesterol (g/L) | 2.25 ± 0.34 | 2.22 ± 0.40 | 0.54 | | |
| | HDL cholesterol (g/L) | 0.61 ± 0.13 | 0.58 ± 0.15 | 0.18 | | |
| | LDL cholesterol (g/L) | 1.42 ± 0.30 | 1.41 ± 0.36 | 0.85 | | |
| | Triglycerids (g/L) | 0.97 (0.76 - 1.25) | 1.05 (0.77 - 1.44) | 0.30 | 1.12 ± 0.70 | 1.14 ± 0.50 |
| | eGFR MDRD (ml/min/1.73m2) | 87.4 ± 16.3 | 90.8 ± 16.1 | 0.13 | | |

| | | | | | | |
|---|---|---|---|---|---|---|
| Values are m±SD or mediane(IQR), % | | | | | | |

### Description:

101 participants of the Stanislas cohort with no LVH at baseline (V1) progressed to LVH at V4. 101 age, gender and SBP matched controls were selected, that did not show LVH neither at V1 nor at V4. No significant differences were found for several other variables of medical history, biology, hemodynamics and demography.

### Conclusion:

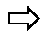 The LVH outcome case control study is suited for the identification of biomarkers associating with the risk of rapid progression to LVH within 5, 10 or 17 years.

### EXAMPLE 2: Association circulating ST2 levels with the risk of progression to LVH in the general population

**Table 2: Crude and adjusted odds ratios for association between LVH at V4 and biomarkers at V1 or V2.**

| Legend: Crude and SBP and eGFR adjusted odds ratios were estimated for the association between biomarker levels (ST2, cTNThs, Mimecan, NTproBNP) at V1 (n=100 no LVH group, n= 100 LVH group), V2 (n=99 no LVH group, n=94 LVH group),V3 (n=57 no LVH group, n=67 LVH group). | | | | | |
|---|---|---|---|---|---|
| | | **Univariate** | | **Multivariate** * | |
| **Biomarker** | | **OR (95% CI)** | **P-value** | **OR (95% CI)** | **P-value** |
| Mimecan at V1 (ng/mL) > 48.59 | | 0.64 (0.33 - 1.24) | 0.190 | 0.63 (0.33 - 1.21) | 0.160 |
| | ST2 at V1 (ng/mL) > 25.51 | 2.21 (1.18 - 4.16) | 0.014 | 2.19 (1.16 - 4.15) | 0.016 |
| cTNThs at V1 (pg/mL) > 2.76 | | 1.60 (0.73-3.53) | 0.240 | 1.8 (0.79-4.10) | 0.160 |
| | Mimecan at V2 (ng/mL) > 50.28 | 1.20 (0.66 - 2.17) | 0.550 | 0.89 (0.48 - 1.67) | 0.720 |
| | ST2 at V2 (ng/mL) > 27.01 | 2.15 (1.12 - 4.16) | 0.022 | 2.15 (1.10 - 4.19) | 0.024 |
| | cTNThs at V2 (pg/mL) > 3.13 | 1.83 (0.91-3.7) | 0.091 | 1.93 (0.93-4.0) | 0.076 |
| | Mimecan at V3 (ng/mL) > 53.62 | 1.75 (0.51 - 5.98) | 0.370 | 1.79 (0.71-4.55) | 0.220 |
| | ST2 at V3 (ng/mL) > 25.06 | 1.67 (0.61 - 4.59) | 0.320 | 1.64 (0.59-4.59) | 0.350 |
| | cTNThs at V3 (pg/mL) >4.09 | 1,86 (0.74-4.65) | 0.190 | 1.79 (0.71-4.55) | 0.220 |
| * adjusted for SBP (ABPM) and eGFR Cut-offs are median | | | | | |

### Description:

Elevated levels of circulating ST2 were found with significant odds ratios in association with LVH outcome. Odds ratios of circulating ST2, but not of circulating Mimecan reached statistical significance at different early points in time (V1 and V2) in association with the LVH outcome variable. Elevated levels of circulating cTNThs were found with significant odds ratios in association with LVH outcome at least at one point in time (V2). Baseline levels of ST2 showed the best prognostic value with regard to progression to LVH within 5, 10 and 17 years. Baseline levels of cTNThs showed moderate prognostic information with regard progression to LVH within 10 years. In contrast baseline levels of mimecan did not show prognostic value with regard to progression to LVH.

### Conclusion:

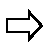 Elevated levels of circulating ST2, but not of Mimecan may help to identify patients at risk of rapid progression to LVH within 5, 10 or 17 years.

### EXAMPLE 3: Association circulating ST2 levels in combination with systolic blood pressure and eGFR with the risk of progression to LVH in the general population

**Table 3: Comparison of nested models between LVH at V4 and biomarkers at V1, V2 or V3 w/o NTproBNP**

| Legend: Mimecan and ST2 were measured in samples of the 202 subjects of the LVH outcome case control study. P-values and likelihood ratio statistics were reported to compare nested models for each biomarker at V1 and V2 w/o NTproBNP. | | |
|---|---|---|
| **Model** | **-2LR** | **P-value** |
| **V1** | | |
| Model M0 : NTproBNP (pg/mL) atV1 + SBP +eGFR | 131.1 | |
| Model M2 : NTproBNP (pg/mL) atV1 + SBP +eGFR + Mimecan at V1 (ng/mL) | 124.558 | 0.16 |
| Model M5 : NTproBNP (pg/mL) atV1 + SBP +eGFR + ST2 at V1 (ng/mL) | 119.431 | 0.0199 |

| **V2** | | |
|---|---|---|
| Model M0 : NTproBNP (pg/mL) atV2 + SBP +eGFR | 125.986 | |
| Model M2 : NTproBNP (pg/mL) atV2 + SBP +eGFR + Mimecan at V2 (ng/mL) | 122.978 | 0.56 |
| Model M5 : NTproBNP (pg/mL) atV2+ SBP +eGFR + ST2 at V2 (ng/mL) | 116.677 | 0.0538 |

Description: It was shown, that model M5 is better than model M0. Elevated circulating ST2 levels were found to improve the association between LVH outcome and NTproBNP compared with NTproBNP alone. In contrast no improvement of model M0 was found with model M2. Mimecan was not found to improve the association between LVH outcome and NTproBNP alone.

### Conclusion:

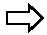 For the identification of subjects at risk to progress to LVH within 5, 10 or 17 years a preferred marker combination comprises NTproBNP, systolic blood pressure, eGFR and ST2.

### EXAMPLE4 : Association of circulating biomarker levels to LVH outcome within 5, 10 or 17 years

**Table 4: Biomarker levels for association between LVH at V4 and biomarkers at V1 or V2.**

| **Biomarker** | **No LVH V1 n=100** | **LVH V1 n=100** |
|---|---|---|
| NTproBNP | 40.9 | 38.1 |
| Mimecan | 49.85 | 47.03 |
| CysC | 0.81 | 0.79 |
| ST2 | 23.58 | 26.82 |

| | **V2 n=100** | **V2 n=100** |
|---|---|---|
| NTproBNP | 43.3 | 51.7 |
| Mimecan | 49.91 | 50.96 |
| CysC | 0.83 | 0.82 |
| ST2 | 24.74 | 27.58 |

| | **V3 n=57** | **V3 n=57** |
|---|---|---|
| NTproBNP | 31.6 | 40.6 |
| Mimecan | 53.42 | 53.81 |
| CysC | 0.80 | 0.82 |
| ST2 | 23.69 | 27.02 |

| | **V4 n=101** | **V4 n=101** |
|---|---|---|
| NTproBNP | 51.20 | 86.02 |
| Mimecan | 59.90 | 62.20 |
| CysC | 0.85 | 0.89 |
| ST2 | 23.40 | 26.24 |

Legend: NTproBNP, Mimecan, CystatinC and ST2 were measured in samples of the 202 subjects of the LVH outcome case control study. Median values were reported to compare elevated levels of each biomarker at V1 (n=100 no LVH group, n= 100 LVH group) V2 (n=99 no LVH group, n=94 LVH group) , V3 (n=57 no LVH group, n=67 LVH group) and V4 (n=101 no LVH group, n=101 LVH group).

### Description:

Elevated levels of circulating ST2, but not of circulating Mimecan, CystatinC or NTproBNP reached statistical significance at V1 (p=0.08, 0.21, 0.23 or 0.85 respectively). No elevated levels of circulating NTproBNP, CystatinC or Mimecan could be found at V1, 17 years prior to LVH outcome. Slight elevated levels of NTproBNP were observed first 10 years before LVH outcome at V3. Elevated levels of NTproBNP reached significance at V4 (p<0.0001), when LVH had become apparent.

Baseline levels of ST2 showed the best prognostic value with regard to progression to LVH within 5, 10 and 17 years. Baseline levels of NTproBNP showed moderate prognostic information with regard progression to LVH within 5 or 10 years. NTproBNP showed the best diagnostic value in association to apparent LVH at V4. In contrast baseline levels of CystatinC or Mimecan did not show any prognostic value with regard to progression to LVH within 5, 10 or17 years.

### Conclusion:

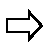 Elevated levels of circulating ST2 may help to identify patients at risk of progression to LVH within 5, 10 or 17 years. Elevated levels of circulating NTproBNP may help to identify patients at risk of rapid progression to LVH within 10 or 5 or 3 years.
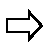 For the identification of subjects at risk to progress to LVH within 5, 10 or 17 years a preferred marker combination comprises ST2 and NTproBNP.
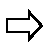 For the identification of subjects at young age (about 40 years) at risk to progress to LVH within 5, 10 or 17 years a preferred marker combination comprises ST2 and NTproBNP.
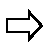 Elevated levels of circulating NTproBNP or cTNThs may help to differentiate the risk when LVH becomes apparent.

## Claims

1. A method for identifying a subject who is at risk of progressing to left ventricular hypertrophy (LVH), comprising the steps of
(a) measuring the amount of the biomarker soluble ST2 (sST2) in a blood, serum or plasma sample obtained from the subject, and
(b) comparing the amount of soluble ST2 to a reference, and
(c) identifying a subject who is at risk of progressing to left ventricular hypertrophy based on the result of the comparison carried out in step (b),
wherein the subject does not suffer from hypertension.

2. The method of claim 1, wherein the subject does not suffer from heart failure, and/or wherein the subject is between 30 and 50 years of age, or wherein the subject is between 40 and 50 years of age, in particular wherein the subject is between 40 and 45 years of age.

3. The method of claims 1 and 2, further comprising the measurement of the amount of NT-proBNP or BNP and/or the amount of a cardiac Troponin in a sample from the subject, and comparing the amount of the NT-proBNP or BNP and/or the amount of the cardiac Troponin to a reference (or to references).

4. The method of any one of claims 1 to 3, further comprising the determination of the estimated glomerular filtration rate (eGFR) in a sample from the subject and comparing the eGFR value to a reference eGFR value.

5. The method of any one of claims 1 to 4, wherein a subject is identified who is at risk of progressing to LVH within a period of 20 years.

6. The method of claim 3, wherein an amount of sST2 above the reference and an amount of NT-proBNP or BNP above the reference is indicative for a subject who is at risk of progressing to LVH within an interval of one month to three years or wherein an amount of sST2 above the reference and an amount of NT-proBNP or BNP below the reference is indicative for a subject who is not at risk of progressing to LVH within an interval of one month to three years.

7. A method for predicting the risk of a subject of progressing to LVH, said method comprising:
(a) measuring the amount of the biomarker soluble ST2 in a blood, serum or plasma sample obtained from a subject who does not suffer from hypertension, and
(b) comparing the amount of soluble ST2 to a reference, and
(c) predicting the risk of the subject of progressing to LVH based on the results of step (b).

8. The method of claim 7, said method further comprising
(A) measuring the amount of NT-proBNP or BNP in the sample, and
(B) comparing the measured amount of step (A) to a reference amount, and
(C) predicting whether the subject is at risk of progressing to LVH within an interval of three years to 20 years,
wherein an amount of sST2 above the reference amount in combination with an amount of NT-proBNP or BNP below the reference amount is indicative for a subject who is at risk of progressing to LVH within an interval of three years to 20 years.

9. *In vitro use* of sST2 or of an agent that specifically binds to sST2 in a blood, serum or plasma sample obtained from a subject for identifying a subject who is at risk of progressing to left ventricular hypertrophy (LVH) or for predicting the risk of a subject of progressing to LVH , wherein the subject does not suffer from hypertension, in particular wherein the agent is an antibody or antigen-binding fragment thereof, wherein said use comprises comparing the amount of soluble ST2 to a reference, and said identifying is based on the result of said comparing.

## Patentansprüche

1. Verfahren zum Identifizieren eines Individuums, bei dem das Risiko besteht, linksventrikuläre Hypertrophie (LVH) zu entwickeln, umfassend die Schritte
(a) Messen der Menge des Biomarkers lösliches ST2 (sST2) in einer Blut-, Serum- oder Plasmaprobe, die von dem Individuum erhalten wurde, und
(b) Vergleichen der Menge an löslichem ST2 mit einer Referenz und
(c) Identifizieren eines Individuums, bei dem das Risiko besteht, linksventrikuläre Hypertrophie zu entwickeln, basierend auf dem Ergebnis des Vergleichs, der in Schritt (b) durchgeführt wurde,
wobei das Individuum nicht an Bluthochdruck leidet.

2. Verfahren nach Anspruch 1, wobei das Individuum nicht an Herzinsuffizienz leidet und/oder wobei das Individuum zwischen 30 und 50 Jahre alt ist oder wobei das Individuum zwischen 40 und 50 Jahre alt ist, insbesondere wobei das Individuum zwischen 40 und 45 Jahre alt ist.

3. Verfahren nach den Ansprüchen 1 und 2, ferner umfassend das Messen der Menge an NT-proBNP oder BNP und/oder der Menge an kardialem Troponin in einer Probe von dem Individuum und das Vergleichen der Menge an NT-proBNP oder BNP und/oder der Menge an kardialem Troponin mit einer Referenz (oder Referenzen).

4. Verfahren nach einem der Ansprüche 1 bis 3, ferner umfassend das Bestimmen der geschätzten glomerulären Filtrationsrate (eGFR) in einer Probe von dem Individuum und das Vergleichen des eGFR-Werts mit einem Referenz-eGFR-Wert.

5. Verfahren nach einem der Ansprüche 1 bis 4, wobei ein Individuum identifiziert wird, bei dem das Risiko besteht, innerhalb eines Zeitraums von 20 Jahren LVH zu entwickeln.

6. Verfahren nach Anspruch 3, wobei eine Menge an sST2 über der Referenz und eine Menge an NT-proBNP oder BNP über der Referenz indikativ für ein Individuum ist, bei dem das Risiko besteht, innerhalb eines Zeitintervalls von einem Monat bis drei Jahren LVH zu entwickeln, oder wobei eine Menge an sST2 über der Referenz und eine Menge an NT-proBNP oder BNP unter der Referenz indikativ für ein Individuum ist, bei dem kein Risiko besteht, innerhalb eines Zeitintervalls von einem Monat bis drei Jahren LVH zu entwickeln.

7. Verfahren zum Vorhersagen des Risikos eines Individuums, LVH zu entwickeln, wobei das Verfahren Folgendes umfasst:
(a) Messen der Menge des Biomarkers lösliches ST2 in einer Blut-, Serum- oder Plasmaprobe, die von einem Individuum erhalten wurde, das nicht an Bluthochdruck leidet, und
(b) Vergleichen der Menge an löslichem ST2 mit einer Referenz und
(c) Vorhersagen des Risikos des Individuums, LVH zu entwickeln, basierend auf den Ergebnissen von Schritt (b).

8. Verfahren nach Anspruch 7, wobei das Verfahren ferner Folgendes umfasst
(A) Messen der Menge an NT-proBNP oder BNP in der Probe und
(B) Vergleichen der gemessenen Menge von Schritt (A) mit einer Referenzmenge und
(C) Vorhersagen, ob bei dem Individuum das Risiko besteht, innerhalb eines Zeitintervalls von drei Jahren bis 20 Jahren LVH zu entwickeln,
wobei eine Menge an sST2 über der Referenzmenge in Kombination mit einer Menge an NT-proBNP oder BNP unter der Referenzmenge indikativ für ein Individuum ist, bei dem das Risiko besteht, innerhalb eines Zeitintervalls von drei Jahren bis 20 Jahren LVH zu entwickeln.

9. In-vitro-Verwendung von sST2 oder eines Mittels, das spezifisch an sST2 bindet, in einer Blut-, Serum- oder Plasmaprobe, die von einem Individuum erhalten wurde, zum Identifizieren eines Individuums, bei dem das Risiko besteht, linksventrikuläre Hypertrophie (LVH) zu entwickeln, oder zum Vorhersagen des Risikos eines Individuums, LVH zu entwickeln, wobei das Individuum nicht an Bluthochdruck leidet, insbesondere wobei das Mittel ein Antikörper oder ein antigenbindendes Fragment davon ist, wobei die Verwendung das Vergleichen der Menge an löslichem ST2 mit einer Referenz umfasst und das Identifizieren auf dem Ergebnis des Vergleichs basiert.

## Revendications

1. Procédé d'identification d'un sujet qui présente un risque de progression vers une hypertrophie ventriculaire gauche (HVG), comprenant les étapes de
(a) mesure de la quantité du biomarqueur ST2 soluble (sST2) dans un échantillon de sang, de sérum ou de plasma obtenu auprès du sujet, et
(b) comparaison de la quantité de ST2 soluble à une référence, et
(c) identification d'un sujet qui présente un risque de progression vers une hypertrophie ventriculaire gauche en se basant sur le résultat de la comparaison réalisée à l'étape (b),
dans lequel le sujet ne souffre pas d'hypertension.

2. Procédé selon la revendication 1, dans lequel le sujet ne souffre pas d'insuffisance cardiaque, et/ou dans lequel le sujet a entre 30 et 50 ans, ou dans lequel le sujet a entre 40 et 50 ans, en particulier dans lequel le sujet a entre 40 et 45 ans.

3. Procédé selon les revendications 1 et 2, comprenant en outre la mesure de la quantité de NT-proBNP ou de BNP et/ou de la quantité d'une troponine cardiaque dans un échantillon provenant du sujet, et la comparaison de la quantité de la NT-proBNP ou du BNP et/ou de la quantité de la troponine cardiaque à une référence (ou à des références).

4. Procédé selon l'une quelconque des revendications 1 à 3, comprenant en outre la détermination du débit de filtration glomérulaire estimé (eGFR) dans un échantillon provenant du sujet et la comparaison de la valeur d'eGFR à une valeur d'eGFR de référence.

5. Procédé selon l'une quelconque des revendications 1 à 4, dans lequel un sujet est identifié qui présente un risque de progression vers une HVG dans une période de 20 ans.

6. Procédé selon la revendication 3, dans lequel une quantité de sST2 au-dessus de la référence et une quantité de NT-proBNP ou de BNP au-dessus de la référence est indicative d'un sujet qui présente un risque de progression vers une HVG dans un intervalle d'un mois à trois ans ou dans lequel une quantité de sST2 au-dessus de la référence et une quantité de NT-proBNP ou de BNP en dessous de la référence est indicative d'un sujet qui ne présente pas de risque de progression vers une HVG dans un intervalle d'un mois à trois ans.

7. Procédé de prédiction du risque de progression d'un sujet vers une HVG, ledit procédé comprenant :
(a) la mesure de la quantité du biomarqueur ST2 soluble dans un échantillon de sang, de sérum ou de plasma obtenu auprès d'un sujet qui ne souffre pas d'hypertension, et
(b) la comparaison de la quantité de ST2 soluble à une référence, et
(c) la prédiction du risque de progression du sujet vers une HVG en se basant sur les résultats de l'étape (b).

8. Procédé selon la revendication 7, ledit procédé comprenant en outre
(A) la mesure de la quantité de NT-proBNP ou de BNP dans l'échantillon, et
(B) la comparaison de la quantité mesurée de l'étape (A) à une quantité de référence, et
(C) la prédiction si le sujet présente un risque de progression vers une HVG dans un intervalle de trois ans à 20 ans,
dans lequel une quantité de sST2 au-dessus de la quantité de référence en combinaison avec une quantité de NT-proBNP ou de BNP en dessous de la quantité de référence est indicative d'un sujet qui présente un risque de progression vers une HVG dans un intervalle de trois ans à 20 ans.

9. Utilisation in vitro de sST2 ou d'un agent qui se lie spécifiquement à sST2 dans un échantillon de sang, de sérum ou de plasma obtenu auprès d'un sujet pour l'identification d'un sujet qui présente un risque de progression vers une hypertrophie ventriculaire gauche (HVG) ou pour la prédiction du risque que présente un sujet de progresser vers une HVG, dans lequel le sujet ne souffre pas d'hypertension, en particulier dans lequel l'agent est un anticorps ou un fragment de liaison à l'antigène de celui-ci, dans lequel ladite utilisation comprend la comparaison de la quantité de ST2 soluble à une référence, et ladite identification est basée sur le résultat de ladite comparaison.
